(19) Europäisches Patentamt

European Patent Office

Office européen des brevets

(11) **EP 1 686 393 A2**

(12) **EUROPEAN PATENT APPLICATION**

(43) Date of publication:
02.08.2006 Bulletin 2006/31

(51) Int Cl.:
*G01S 7/52* (2006.01)          *G01S 15/89* (2006.01)
*G10K 11/34* (2006.01)

(21) Application number: 05026184.1

(22) Date of filing: 15.12.2005

(84) Designated Contracting States:
AT BE BG CH CY CZ DE DK EE ES FI FR GB GR
HU IE IS IT LI LT LU LV MC NL PL PT RO SE SI
SK TR
Designated Extension States:
AL BA HR MK YU

(30) Priority: 27.01.2005 US 46347

(71) Applicant: Siemens Medical Solutions USA, Inc.
Malvern, PA 19355-1406 (US)

(72) Inventors:
• Bradley, Charles E.
Burlingame, CA 94010 (US)

• Lazenby, John C.
Fall City, WA 98024 (US)
• Liu, D-L Donald
Issaquah, WA 98027 (US)
• Thomas, Lewis J.
Palo Alto, CA 94306 (US)
• Ustuner, Kutay F.
Mountain View, CA 94041 (US)

(74) Representative: Hazzard, Alan David
Siemens AG
Postfach 22 16 34
80506 Munich (DE)

(54) **Coherence factor adaptive ultrasound imaging**

(57)     A set of NxM signals are acquired (40) from an object, where N is the number of array (12) elements and M corresponds to variations in data acquisition and/or processing parameters. The parameters include transmit aperture functions, transmit waveforms, receive aperture functions, and receive filtering functions in space and/or time. A coherence factor is computed (52) as a ratio of the energy of the coherent sum (44) to the energy of the at-least-partially incoherent sum (46) of channel or image signals acquired with at least one different parameter. Partial beamformed data may be used for channel coherence calculation. For image domain coherence, a component image (40) is formed for each different transmit beam or receive aperture function, and a coherence factor image is computed using the set of component images. The coherence factor image is displayed or used to modify or blend other images formed of the same region.

FIG.2

## Description

## BACKGROUND

This present invention relates to adapting ultrasound imaging as a function of coherence. In particular, imaging is performed as a function of the coherence of acquired data.

Lack of coherence may be limited by aberration correction and array calibration. Aberration correction suffers from various problems, such as the lack of suitable point targets, aberration is propagation path-dependent and varies with time due to tissue and transducer movement, and estimation and correction requires fine spatial sampling and high computational costs. Array calibration is not commonly used in practice due to complexity of software and system-probe integration.

Clutter may be suppressed using a coherence factor. For example, U.S. Patent No. _____, (Application Serial No. 10/814,959, filed March 31, 2004), the disclosure of which is incorporated herein by reference, discloses use of a measure of coherence. The coherence factor is computed using channel data received in response to a focused transmit beam. For random scatterers and in the absence of propagation aberration, the coherence of channel data is inversely proportional to the transmit beam width. Therefore, the most coherent echoes are usually returned from the transmit focal depth. However, when the transmit beam is broad or unfocused, such as at depths shallower or deeper than the transmit focus, the coherence factor computed using waveforms received by individual elements is low irrespective of the degree of aberration and clutter, and may not be useful to discriminately suppress clutter relative to real targets.

Broad transmit beams are increasingly being used to gain scan speed. However, due to the weak or non-existing transmit focus, clutter levels are usually higher compared to conventional imaging that uses focused transmit beams. This clutter is attributed to tissue aberration, array nonuniformities, and beamforming quantization effects.

## BRIEF SUMMARY

By way of introduction, the preferred embodiments described below include a method and systems for adaptive ultrasound imaging. Consider a set of NxM signals acquired from an object, where N is the number of array elements and M corresponds to variations in data acquisition and/or processing parameters. The data acquisition and processing parameters include transmit aperture functions, transmit waveforms, receive aperture functions, and receive filtering functions in space and/or time. A coherence factor is computed as a ratio of the energy of the coherent sum to the energy of the at-least-partially incoherent sum of channel or image signals acquired with at least one different parameter.

In one embodiment, a component image is formed for each different transmit beam or receive aperture function, and a coherence factor image is computed using the set of component images. The coherence factor is calculated from data in the image domain rather than using the coherent and incoherent sum of channel data.

In a first aspect, a method is provided for adaptive ultrasound imaging. First and second frames of image domain data are obtained. Both the first and second frames represent a plurality of locations in a scanned region. A coherence factor is determined as a function of the image domain data from the first and second frames. Information is generated as a function of the coherence factor.

In a second aspect, a method is provided for adaptive ultrasound imaging. First and second broad transmit beams are transmitted. First and second sets of data are obtained in response to the first and second broad transmit beams, respectively. The sets correspond to channel or image domain data. The first set of data is obtained as a function of a different transmit aperture function, transmit waveforms, receive aperture functions, receive filtering function, or combinations thereof in space, time or both space and time than the second set of data. A coherence factor is determined as a function of the first and second sets of data.

The present invention is defined by the following claims, and nothing in this section should be taken as a limitation on those claims. Further aspects and advantages of the invention are discussed below in conjunction with the preferred embodiments.

## BRIEF DESCRIPTION OF THE DRAWINGS

The components and the figures are not necessarily to scale, emphasis instead being placed upon illustrating the principles of the invention. Moreover, in the figures, like reference numerals designate corresponding parts throughout the different views.

Figure 1 is a block diagram of one embodiment of a system for adaptive ultrasound imaging as a function of a coherence factor; and

Figure 2 is a flow chart diagram of one embodiment of a method for adaptive ultrasound imaging as a function of a coherence factor.

## DETAILED DESCRIPTION OF THE DRAWINGS AND PRESENTLY

## PREFERRED EMBODIMENTS

A set of NxM signals are acquired from an object, where N is the number of array elements and M corresponds to variations in data acquisition and processing parameters. These parameters encompass transmit aperture functions, transmit waveforms, receive aperture functions, and receive filtering functions in both space and time. A coherence factor is computed as a ratio of the energy of the coherent sum to the energy of the at-least-partially incoherent sum of these signals. In one embodiment, a coherence factor image is computed in the image domain as a function of the coherent and incoherent summations of the component images formed with different parameters. At least one parameter is modified as a function of the coherence factor. In one embodiment coherence factor is used to modulate the gray level or color of the image synthesized using the component images.

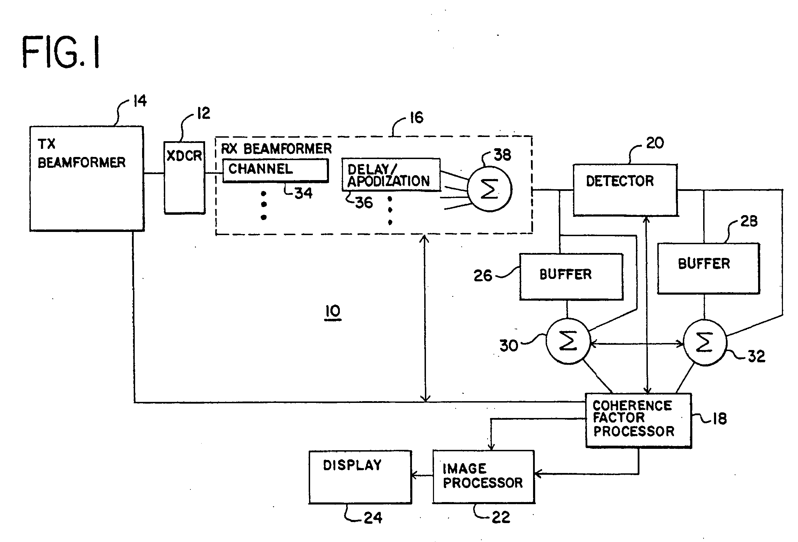

Figure 1 shows one embodiment of a system 10 for adaptive ultrasound imaging. The system 10 is an ultrasound imaging system, but other imaging systems using multiple transmit or receive antennas (i.e., elements) may be used. The system 10 includes a transducer 12, a transmit beamformer 14, a receive beamformer 16, a coherence factor processor 18, a detector 20, an image processor 22, a display 24, buffers 26, 28 and summers 30, 32. Additional, different or fewer components may be provided, such as a system 10 without the display 24.

The transducer 12 is an array of a plurality of elements. The elements are piezoelectric or capacitive membrane elements. The array is configured as a one-dimensional array, a two-dimensional array, a 1.5D array, a 1.25D array, a 1.75D array, an annular array, a multi-dimensional array, combinations thereof or any other now known or later developed array. The transducer elements transduce between acoustic and electric energies. The transducer 12 connects with the transmit beamformer 14 and the receive beamformer 16 through a transmit/receive switch, but separate connections may be used in other embodiments.

Two different beamformers are shown in the system 10, a transmit beamformer 14 and the receive beamformer 16. While shown separately, the transmit and receive beamformers 14, 16 may be provided with some or all components in common. Both beamformers connect with the transducer array 12.

The transmit beamformer 14 is a processor, delay, filter, waveform generator, memory, phase rotator, digital-to-analog converter, amplifier, combinations thereof or any other now known or later developed transmit beamformer components. In one embodiment, the transmit beamformer 14 is the transmit beamformer disclosed in U.S. Patent No. 5,675,554, the disclosure of which is incorporated herein by reference. The transmit beamformer is configured as a plurality of channels for generating electrical signals of a transmit waveform for each element of a transmit aperture on the transducer 12. The waveforms are unipolar, bipolar, stepped, sinusoidal or other waveforms of a desired center frequency or frequency band with one, multiple or fractional number of cycles. The waveforms have relative delay or phasing and amplitude for focusing the acoustic energy. The transmit beamformer 14 includes a controller for altering an aperture (e.g. the number of active elements), an apodization profile across the plurality of channels, a delay profile across the plurality of channels, a phase profile across the plurality of channels, center frequency, frequency band, waveform, shape, number of cycles and combinations thereof. A scan line focus is generated based on these beamforming parameters. -Alteration of the beamforming parameters may correct for aberrations or clutter.

The receive beamformer 16 is a preamplifier, filter, phase rotator, delay, summer, base band filter, processor, buffers, memory, combinations thereof or other now known or later developed receive beamformer components. In one embodiment, the receive beamformer is one disclosed in U.S. Patent Nos. 5,555,534 and 5,685,308, the disclosures of which are incorporated herein by reference. The receive beamformer 16 is configured into a plurality of channels 34 for receiving electrical signals representing echoes or acoustic energy impinging on the transducer 12. Beamforming parameters including a receive aperture (e.g., the number of elements and which elements used for receive processing), the apodization profile, a delay profile, a phase profile, frequency and combinations thereof are applied to the receive signals for receive beamforming. For example, relative delays and amplitudes or apodization focus the acoustic energy along one or more scan lines. A control processor controls the various beamforming parameters for receive beam formation. Beamformer parameters for the receive beamformer 16 are the same or different than the transmit beamformer 14. For example, an aberration or clutter correction applied for receive beam formation is different than an aberration correction provided for transmit beam formation due to difference in signal amplitude.

Figure 1 shows one possible embodiment of the receive beamformer 16. A channel 34 from each of the elements of the receive aperture within the array 12 connects to an amplifier and/or delay 36 for applying apodization amplification. An analog-to-digital converter digitizes the amplified echo signal. The digital radio frequency received data is demodulated to a base band frequency. Any receive delays; such as dynamic receive delays and/or phase rotations are then applied by the amplifier and/or delay 36. The receive beamformer delayed or phase rotated: base band data for each channel may be provided to a buffer for channel based coherence determinations. The buffer is sufficient to store digital samples of the receive beamformer 16 across all or a portion of the receive aperture from a given range. The beamform summer 38 is one or more digital or analog summers operable to combine data from different channels 34 of the receive aperture to form one or a plurality of receive beams. The summer 38 is a single summer or cascaded summer. The summer 38 sums the relatively delayed and apodized channel information together to form a beam. In one embodiment, the beamform summer 38 is operable to sum in-phase and quadrature channel data in a complex manner such that phase information is maintained for the formed beam. Alternatively, the beamform summer sums data amplitudes or intensities without maintaining the phase information.

In one embodiment, the transmit beamformer 14 and receive beamformer 16 operate using broad beam transmission. For example, the transmit beamformers and/or receive beamformers disclosed in U.S. Patent No. 6,685,641, the disclosure of which is incorporated herein by reference, is used. The receive beamformer 16 using a transform to generate image data or alternatively sequentially or in parallel forms a plurality of receive beams in response to the broad transmit beam. Broad beam transmissions include unfocused or weakly focused ultrasonic waves that insonify a region, such as a majority of a two dimensional region to be scanned, from one or more angles. A virtual point source may be used at a large or substantially infinite distance behind an array to define a broad transmit beam. The virtual point source may be moved laterally relative to the array to steer the broad transmit beam. To compensate for undesired divergence, a mildly focused planar wave is generated as the broad transmit wavefront. The energy generated by each element of the transducer array 12 is delayed relative to other elements to steer or mildly focus a plane wave. A Gaussian or hamming apodization function is applied across the transducer array 12 to reduce edge waves generated by the finite aperture provided by the transducer array 12. Since no specific transmit focal points are specified, dynamic transmit focusing is realized by the superposition of transmitting plane waves at different angles to the transducer array 12. Other techniques for generating plane waves, such as using other types of apodization or using a mildly diverging plane wave may be used.

The receive beamformer 16 outputs image data, data representing different spatial locations of a scanned region. The image data is coherent (i.e., maintained phase information), but may include incoherent data. The data may be formed by processing received data, such as synthesizing scan lines (i.e., coherent combination), compounding data from multiple scan lines (i.e., incoherent combination) or other processes for generating data used to form an image from received information. For example, inter-beam phase correction is applied to one or more beams and then the phase corrected beams are combined through a coherent (i.e., phase sensitive) filter to form synthesized ultrasound lines and/or interpolated between beams to form new ultrasound lines. Once the channel data is beamformed or otherwise combined to represent spacial locations of the scanned region, the data is converted from the channel domain to the image data domain.

The detector 20 is a general processor, digital signal processor, application-specific integrated circuit, control processor, digital circuit, summer, filter, finite impulse response processor, multipliers, combinations thereof or other now known or later developed processors for forming incoherent image data from received signals. The detector 20 includes a single or multiple processors with or without log compression. The detector 20 detects the amplitude, intensity, log-compressed amplitude or power of the beamformed signals. For example, the detector 20 is a B-mode detector. One or more filters, such as spatial or temporal filters may be provided with the detector 20. The detector 20 outputs incoherent image data.

The buffers 26 and 28 are first-in, first-out buffers, memories, corner-turning memories or other now known or later developed memories for storing image data. Each buffer 26, 28 is operable to store one or more data values representing one or more scanned locations. For example, the buffers 26, 28 each store data associated with an entire scan. One buffer 26 is operable to store coherent image data, and the other buffer 28 is operable to store incoherent image data. A same buffer 26, 28 may be used to store both the incoherent and coherent data. The buffers 26, 28 store the data from a previous scan, such as an immediately previous scan. Additional buffers 26, 28 may be used for storing data from more than one previous scan.

The summers 30, 32 are digital or analog summers, processors, a same processor, summing nodes, logic devices, the coherence factor processor 18 or other now known or later developed summer. The summers 30, 32 sum image data representing a same or substantially same spatial location, but responsive to a different transmit aperture functions (apodization, delay profile, aperture position, aperture shape or aperture size), transmit waveforms, receive aperture functions, and/or receive filtering functions. A transmit or receive parameter is altered between two sets of image data. The buffers 26, 28 store the earlier set of image data while the subsequent set of image data is acquired. The two sets are then combined. The summer 30 combined the sets coherently with the maintained phase information. The summer 32 combines the sets incoherently.

The coherence factor processor 18 determines an amount of coherence of the data in the image domain from the outputs of the summers 30, 32. The coherence factor processor 18 is a general processor, digital signal processor, control processor, application specific integrated circuit, digital circuit, digital signal processor, analog circuit, combinations thereof or other now known or later developed processors for controlling the transmit beamformer 14, the receive beamformer 16, the detector 20, the image processor 22 or other components of the system 10. In one embodiment, the coherence factor processor 18 is the beamformer or system controller, but a separate or dedicated processor may be used in other embodiments. The coherence factor processor 18 is operable to determine a coherence factor as a function of ultrasound image data. The coherence factor is calculated for one or for a plurality of the spatial locations represented by the image data. For example, a coherence factor value is calculated for each of the spatial locations within an overlapping scanned region. Additionally or alternatively, the coherence factor processor 18 connects with the receive beamformer 16 and a buffer for obtaining delayed or phase rotated channel data from each of the channels of a receive aperture for determining coherence in the channel domain.

The coherence factor processor 18 may include a low-pass filter for determining a low-passed filtered coherence factor as a function of time or space. For example, the coherence factors for an overlapping scanned region are low pass filtered to reduce spatial variation. The coherence factor processor 18 may include a detector or path for routing coherently combined data to the detector 20. The coherently combined data is detected and used for imaging.

The coherence factor processor 18 is operable to determine a beamforming parameter, image forming parameter, or image processing parameter for adaptive imaging as a function of the coherence factor. Parameters are then adaptively altered to reduce side lobe clutter in an eventual image. Any of the beamformer parameters used by the transmit beamformer 14, the receive beamformer 16, the detector 20, and/or the image processor 22 may be responsive to a coherence factor calculated by the coherence factor processor 18. Adaptive imaging is additionally or alternatively provided by generating an image as a function of the coherence factor, such as generating an image representing the coherence factor.

The image data is output to the image processor 22. The image processor 22 is operable to set a display dynamic range, filter in space and time using a linear or nonlinear filter which may be an FIR or IIR filter or table-based, and map the signal amplitude to display values as a function of a linear or non-linear map. The non-linear map may use any of various inputs, such as both filtered and unfiltered versions of the data being input in selecting a corresponding brightness. Data optimized for contrast may be input with the same or similar data optimized for spatial resolution. The input data is then used to select brightness or display intensity. The image processor 22 scan converts the data and outputs the data as an one-, two-, or three-dimensional representation on the display 24. Since one of the beamforming parameters, image forming parameters, dynamic range, non-linear mapping, non-linear filtering or combinations thereof is selected or altered as a function of the coherence factor, the resulting image more likely shows the desired targets without artifacts from side lobe contributions. For example, the coherence factor is used to adaptively alter parameters for subsequent imaging, such as applying coherence factor for adjusting aberration corrections for beamforming parameters, and adjusting the type or amount of synthesis and compounding performed by the image forming processor 20. In another embodiment, the image processor 22 generates an image representing the coherence factor, such as modulating color, hue, brightness, shade or other imaged value as a function of the coherence factor.

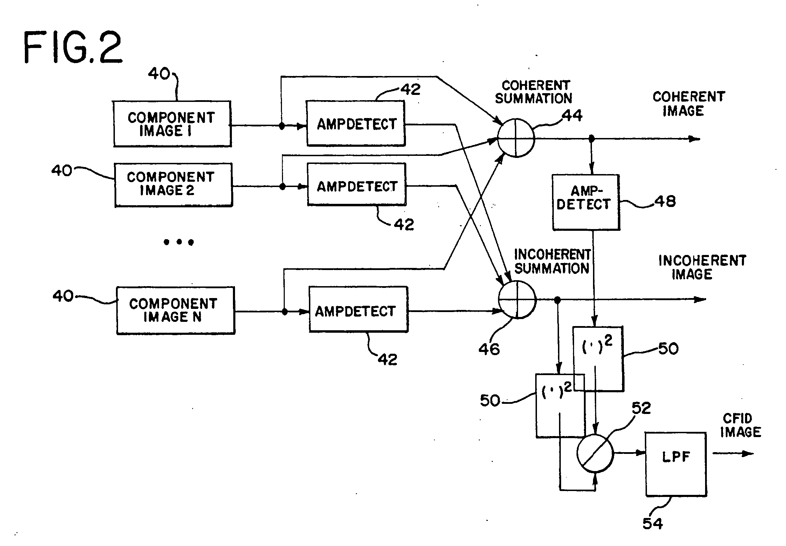

Figure 2 shows a method for adaptive ultrasound imaging. The method is implemented by the system 10 of Figure 1 or a different system. Additional, different or fewer acts may be provided. For example, acts 48, 50 and/or 54 are not provided. As another example, additional or alternative acts for determining coherence factor for channel data are provided, such as disclosed in U.S. Patent No.                 , (Application Serial No. 10/814,959, filed March 31, 2004), the disclosure of which is incorporated herein by reference. The coherence factor for channel or image data is determined from two or more sets of data acquired in response to different parameter values.

In act 40, two or more frames of image domain data are obtained. In one embodiment, each frame of image domain data is acquired in response to transmission of a respective broad transmit beam. Each of the broad transmit beams covers a majority of a two-dimensional plane across a scanned region. Alternatively, a lesser area is covered. A single broad transmit beam may allow formation of an image of the entire region of interest, resulting in a high frame rate. Alternatively, multiple transmissions to different areas scan an entire region of interest. The scan is for two or three-dimensional imaging. The broad transmit beam may extend along two or three-dimensions.

In response to the transmissions, channel data is received. The frames of image domain data are formed by summing channel data from every element of a receive aperture together for each of the plurality of locations. Alternatively, transforms, such as a Fourier transform, or other processes are applied to the channel data to generate image data representing spatial locations of the scanned region. The image data includes values representing a plurality of locations in a scanned region. Frames of data include data sets associated with a particular scan or combinations of scans. A frame of data includes data representing the region of interest whether or not the data is transmitted in a frame format.

Partial beamforming or beamsums may be used. For example, sub-array beamforming with a multi-dimensional transducer array is provided to limit the number of cables to connect the array with an imaging system. The partial beamsummed data for each sub-array is treated as channel data. The channel data is beamformed together for determining the coherence factor from image domain data. Alternatively, the partial beamsums are used as channel data to determine the coherence factor in the channel domain.

The obtained image data includes phase information. Image data is in a radio frequency (RF) or in-phase and quadrature (IQ) format. Each set or frame of image data may be denoted mathematically as $S_n(xy)$, where $(x,y)$ is a point in the image and n represents a specific transmit and receive function settings .

The different sets or frames of image data are responsive to different transmit aperture functions, transmit waveforms, receive aperture functions, receive filtering functions, or combinations thereof. The differences in the parameters or function settings are in space, time or both space and time. For example, the transmit aperture function varies as a function of virtual point sources at different positions. A first set of image data is obtained with a broad transmit beam transmitted at a first angle relative to the array, and a second set of image data is obtained with a broad transmit beam transmitted at a second, different angle relative to the array. In one embodiment, eleven component images or frames of data are acquired with plane wave incident angles from - 10 to 10 degrees in 2 degrees steps. As another example, the receive aperture function varies to use different portions or apodization of an array. The receive aperture shape or position is altered between acquisition of the two or more different sets of image data. As yet another example, the receive filtering function varies temporally. A first frame of data is acquired at a different frequency band than the second frame of data, such as a fundamental frequency band for one set and a harmonic frequency band for the other set. As another example, the receive filtering varies spatially. The first frame of data is acquired along a different viewing direction or filtering direction than the second frame. Other functions, parameters, variables or settings may be adjusted or varied between acquisitions of the two or more component frames of data.

The obtained sets of data represent two-dimensional images or regions. Sets of data representing three-dimensional images or volumes may be used.

In act 42, the frames of coherent image data are detected. Amplitude, intensity, power or other characteristics of the signals are detected. The detection may be in the log domain or without log compression. Amplitude detection may be done in a number of ways, such as using the Hilbert transform, or demodulation to IQ signals along the axial direction and using $\sqrt{I^2 + Q^2}$ as the amplitude. Detection removes the phase information, resulting in incoherent data.

In act 44, the two or more frames of image domain data are summed. Values representing the same or substantially same location are coherently summed. The phase relationship between the data is maintained. The coherent sum of the component images yields a coherent image:

$$S(x, y) = \frac{1}{N} \sum_{n=1}^{N} S_n(x, y)$$

The sum is an average, true sum, weighted summation or another combination function. Linear or non-linear combination may be provided.

In act 46, the two or more frames of image domain data are summed again. Values representing the same or substantially same location are incoherently summed. Detected data or incoherent data is summed, such as summing in the amplitude, intensity or log domains. The same sets of data used in act 44, but with detection, or different sets of data are used in act 46. Amplitude detecting in act 42 provides frames of incoherent image data, $A_n(x,y)$. The component amplitude images are summed together:

$$A(x, y) = \frac{1}{N} \sum_{n=1}^{N} A_n(x, y)$$

The sum is an average, true sum, weighted summation or another combination function. Linear or non-linear combination may be provided.

In act 48, the coherently summed image domain data is amplitude detected. Th detection is the same or different than performed in act 42. The detection provides image domain data output from act 44 in a same format or subject to similar processes as the image domain data output from act 46. The data is different due to the coherent summation as opposed to incoherent summation.

In act 50, additional optional operations are performed prior to determining a coherence factor. For example, both the coherently and incoherently summed image data is squared.

In act 52, the coherence factor is determined as a function of the first and second sets of data or image domain data from the first and second frames. The coherence factor values are determined for each of the plurality of locations, such as the entire scanned region of interest. Alternatively, a single coherence factor value is calculated. In yet other embodiments, one or more coherence factor values are each calculated from data representing more than one spatial location.

The coherence factor is calculated as a function of the coherent and incoherent sums. For example, the coherence factor is a ratio of energy of a coherent sum to energy of an incoherent sum. Let $B(x,y)$ be the result of amplitude detection of $S(x,y)$, the coherence factor is computed as:

$$\text{CFID}(x, y) = \frac{B^2(x, y)}{A^2(x, y)}$$

where B is coherent amplitude and A is incoherent amplitude. The coherence factor will fall within the range of 0 to 1. Other functions for calculating coherence of image data may be used, such as without squaring the amplitudes, or doing the computation as a difference in the log domain.

The coherent image $B(x,y)$ usually has higher lateral resolution and larger speckle variance than the incoherent image $A(x,y)$. The amplitude variations of the coherence factor values representing different spatial locations (coherence factor image) may be smoothed in act 54. Spatial low-pass filtering may suppress or limit the speckle in the coherence factor image. The filtered coherence factor image may be used to modify other images without introducing or enhancing speckles. The low-pass filtering also averages the coherence factors locally to improve the accuracy and reduce the variance of the calculated coherence factor.

The coherence factor, whether from channel or image domain calculations, is used to generate information. For example, the transmit or receive parameters are adjusted as a function of the coherence factor, such as disclosed in U.S. Patent No. _____, (Application Serial No. 10/814,959, filed March 31, 2004). The coherence factor image is used to modify subsequent image formation. For example, partial beamsum signals are used to calculate channel based coherence. The coherence factor is then used to alter transmit or receive beamformation or filtering functions.

The coherence factor information or image may be used to weight or modify the component images, the coherently summed image, the incoherently summed image, subsequent image information, images obtained separately with broad or focused transmit beams, combinations thereof or other image information. Brightness, color, hue, shade or combinations thereof of detected data is modulated as a function of the coherence factor. For example, the weighting can be performed in the linear amplitude domain,

$$O(x,y) = I(x,y) \times f\,[\text{CFID}(x,y)]$$

where $f(u)$ is in general some non-linear function, $I(x,y)$ is the input image, and $O(x,y)$ is the output image. If $f(u) = u,$ then this operation reduces the image amplitude in proportion to the coherence factor at each image location. A thresholding function for $f(u)$ may limit the dynamic range of gain suppression.

Incoherent summation (compounding) may suppress speckle and improve boundary depiction. The component images are formed using different transmit steering with possibly different receive aperture selection schemes, such as partially overlapping sub-apertures. Coherent factor information is computed using the component images corresponding to each transmit/receive aperture scheme. This coherence factor image can then be used to modulate the compounded image in brightness or color.

Other than the coherent and incoherent images, composite images can also be formed from individual component images. Some component images are added together coherently, and the sums are then added incoherently. Generally, adding component images coherently helps improve lateral resolution and provides redundancy in image formation which helps reducing clutter from various sources. Adding images incoherently helps reduce speckle. The transducer and tissue motion is limited so that motion during the acquisition of individual component images is small compared to the acoustic wavelength.

In one embodiment, an image is displayed as a function of the coherence factor. The coherence factor information or a function of the image is displayed along with gray-scale images. For example, a color overlay of coherence factor is displayed on top of gray-scale B-mode images. This registers the coherence factor image on the gray-scale image, and helps identify which part of tissue is introducing more inhomogeneity in wave propagation.

In another embodiment, the first and second sets of data or frames of data are blended as a function of the coherence factor. The coherence factor or a function of the coherence factor is used to selectively blend two images together to produce an output image. For example, two input images are the coherently summed image and the incoherently summed image. The coherence factor determines the relative weight in combining the summed images. If the coherence is high, the incoherently summed imaging information is more heavily weighted. Another example is to blend the coherent image and a low-pass filtered version of the coherent image as a function of the coherence factor.

A spatial average of the coherence factor over a region indicates the degree of tissue aberration. The degree of aberration serves as an image quality index which may provide useful information in diagnosis. For example, in breast imaging, if a patient's image has a lower average coherence factor, the imaged tissue is likely to be more inhomogeneous. Inhomogeneous tissue may be related to tissue pathology. In phantom studies, an average of coherence factor was found to be 0.61 with no aberration and 0.41 with aberration.

Where tissue aberration is severe, the component image samples at that location and all other subsequent locations away from the transmit aperture are more likely to be out of phase. Lower coherence factor values are expected at such locations. The derivative of the coherence factor information along the insonification angle approximates the degree of local tissue aberration. The coherence factor information without the derivative is a measure of the aberration integrated along the insonification path.

While the invention has been described above by reference to various embodiments, it should be understood that many changes and modifications can be made without departing from the scope of the invention. It is therefore intended that the foregoing detailed description be regarded as illustrative rather than limiting, and that it be understood that it is the following claims, including all equivalents, that are intended to define the spirit and scope of this invention.

**Claims**

1. A method for adaptive ultrasound imaging, the method comprising:

obtaining (40) at least first and second frames of image domain data, both the first and second frames representing a plurality of locations in a scanned region;

determining (52) a coherence factor as a function of the image domain data from the first and second frames; and

generating information comprising image data, a beamforming parameter, an image forming parameter, an image processing parameter or combinations thereof as a function of the coherence factor.

2. The method of Claim 1 wherein obtaining (40) the first and second frames comprises transmitting first and second broad transmit beams, respectively, each of the first and second broad transmit beams covering an overlapping region of a two-dimensional plane or a three-dimensional volume across the scanned region.

3. The method of Claim 1 wherein obtaining (40) the first and second frames of image domain data comprises summing channel data from every element of a receive aperture together for each of the plurality of locations, the summed channel data being image domain data.

4. The method of Claim 1 wherein obtaining (40) the first and second frames of image domain data comprises forming (40) data representing each of the plurality of locations from channel data as a function of a Fourier transform.

5. The method of Claim 1 wherein obtaining (40) the first and second frames of image domain data comprises obtaining (40) the first frame of image domain data in response to a different transmit aperture function, transmit waveforms, receive aperture functions, receive filtering function, or combinations thereof in space, time or both space and time than the second frame of image domain data.

6. The method of Claim 5 wherein the transmit aperture function including apodization and delay profile varies as a function of virtual point sources at different positions.

7. The method of Claim 5 wherein the receive aperture function including apodization and delay profile varies to use different portions or apodization of an array.

8. The method of Claim 5 wherein the receive filtering function varies temporary, the variation operable to provide the first frame of data at a different frequency band than the second frame of data.

9. The method of Claim 5 wherein the receive filtering varies spatially, the variation operable to provide the first frame along a different viewing direction than the second frame.

10. The method of Claim 1 wherein determining (52) the coherence factor comprises determining (52) coherence factor values for each of the plurality of locations.

11. The method of Claim 1 wherein determining (52) the coherence factor comprises:

summing (44) the first and second frames of image domain data coherently;

summing (46) the first and second frames of image domain data at least partially incoherently;

calculating (52) the coherence factor as a function of the coherent and incoherent sums.

12. The method of Claim 11 wherein summing (46) incoherently comprises summing (46) in an amplitude, intensity or log domains.

13. The method of Claim 1 wherein generating information as a function of the coherence factor comprises displaying an image as a function of the coherence factor for each of the plurality of locations.

14. The method of Claim 1 wherein generating information as a function of the coherence factor comprises modifying brightness, color, hue, shade or combinations thereof of the first frame of data, the second frame of data, a third frame of data, a frame of data from an incoherent sum, a frame of data from a coherent sum or combinations thereof as a function of the coherence factor.

15. The method of Claim 1 wherein generating information as a function of the coherence factor comprises blending the first and second frames of data as a function of the coherence factor.

16. A method for adaptive ultrasound imaging, the method comprising:

transmitting first and second broad transmit beams;

obtaining (40) first and second sets of data in response to the first and second broad transmit beams, respectively, the first set of data obtained as a function of a different transmit aperture function, transmit waveforms, receive aperture functions, receive filtering function, or combinations thereof in space, time or both space and time than the second set of data; and

determining (52) a coherence factor as a function of the first and second sets of data.

**17.** The method of Claim 16 wherein the coherence factor is a ratio of energy of a coherent sum to energy of an incoherent sum of the first and second sets of data.

**18.** The method of Claim 16 wherein obtaining (40) comprises obtaining (40) first and second frames of image domain data, both the first and second frames representing a plurality of locations in a scanned region.

**19.** The method of Claim 16 wherein obtaining (40) comprises obtaining (40) as a function of the transmit aperture function, the transmit aperture function varying as a function of virtual point sources at different positions for the first and second sets of data.

**20.** The method of Claim 16 wherein obtaining (40) comprises obtaining (40) as a function of the receive aperture function, the receive aperture function varying to use different portions or apodization of an array for the first and second sets of data.

**21.** The method of Claim 16 wherein obtaining (40) comprises obtaining (40) as a function of the receive filtering function, the receive filtering function varying temporally to provide the first set of data at a different frequency band than the second set of data.

**22.** The method of Claim 16 wherein obtaining (40) comprises obtaining (40) as a function of the receive filtering function, the receive filtering function varying spatially to provide the first set of data along a different viewing direction than the second set of data.

**23.** The method of Claim 16 wherein determining (52) the coherence factor comprises:

summing (44) the first and second frames of image domain data coherently;
summing (46) the first and second frames of image domain data at least partially incoherently;
calculating (52) the coherence factor as a function of the coherent and incoherent sums.

**24.** The method of Claim 16 further comprising:

displaying an image as a function of the coherence factor.

**25.** The method of Claim 16 further comprising:

modifying image brightness, color, hue, shade or combinations thereof of as a function of the coherence factor.

**26.** The method of Claim 16 further comprising:

blending the first and second sets of data weighted as a function of the coherence factor.

**27.** The method of Claim 16 wherein obtaining (40) comprises forming a plurality of partial beamsums and wherein determining the coherence factor comprises determining the coherence factor as a function of the partial beamsums.

# FIG. 1

EP 1 686 393 A2

# FIG.2